(19) Europäisches Patentamt
European Patent Office
Office européen des brevets

(11) Numéro de publication: **0 061 379**
**B1**

(12) # FASCICULE DE BREVET EUROPEEN

(45) Date de publication du fascicule du brevet: **02.10.85**

(51) Int. Cl.[4]: **C 07 D 213/69**, A 61 K 31/44 // C07D213/65, C07D405/12

(21) Numéro de dépôt: **82400397.4**

(22) Date de dépôt: **08.03.82**

(54) **Dérivés de pyridine, leur préparation et leur application en thérapeutique.**

(30) Priorité: **25.03.81 FR 8106005**

(43) Date de publication de la demande: **29.09.82 Bulletin 82/39**

(45) Mention de la délivrance du brevet: **02.10.85 Bulletin 85/40**

(84) Etats contractants désignés: **AT BE CH DE FR GB IT LI LU NL SE**

(56) Documents cités: **FR - A - 2 218 101**

**Le dossier contient des informations techniques présentées postérieurement au dépôt de la demande et ne figurant pas dans le présent fascicule.**

(73) Titulaire: **SYNTHELABO, 58, rue de la Glacière, F-75621 Paris Cedex 13 (FR)**

(72) Inventeur: **Manoury, Philippe, 9, rue de Malabry, F-92350 Le Plessis Robinson (FR)**
Inventeur: **Binet, Jean, 12 Hameau de la Gondole, F-91650 Breuillet (FR)**
Inventeur: **Cavero, Icilio, 8, rue Gabriel Fauré, F-94000 Créteil (FR)**

(74) Mandataire: **Thouret-Lemaitre, Elisabeth et al, Service Brevets - SYNTHELABO 58, rue de la Glacière, F-75621 Paris Cedex 13 (FR)**

EP 0 061 379 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

BUNDESDRUCKEREI BERLIN

**Description**

La présente invention a pour objet des dérivés de pyridine, leur préparation et leur application en thérapeutique.

Des dérivés de pyridine à activité $\beta$-bloquante ont déjà été décrits dans la littérature, par exemple dans le brevet français n° 74 05 391 (2 218 101).

Les composés de l'invention diffèrent toutefois de ces composés par leur structure et leur très bonne activité thérapeutique.

Les composés de l'invention répondent à la formule (I)

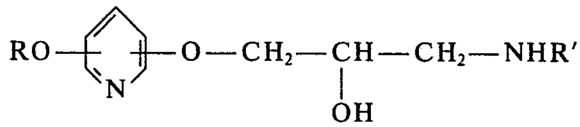

(I)

dans laquelle

- R représente un radical $R_1O(CH_2)_2$ — dans lequel
  $R_1$ est un radical $(C_{3-6})$cycloalkyle, un radical $(C_{3-6})$cycloalkyl-$(C_{1-4})$alkyle ou un radical allyle, et
- R' représente un radical isopropyle ou tertiobutyle.
  Les composés de l'invention comportent un carbone asymétrique. Les racémates et les antipodes levogyres et dextrogyres font partie de l'invention.

Les composés préférés de l'invention sont ceux dans lesquels le groupe $-O-CH_2-CHOH-CH_2-NHR'$ est en position 2 sur le cycle pyridinique, et parmi ceux-ci les composés pour lesquels RO est en position 5.

Les radicaux R sont de préférence les radicaux $[(C_{3-6})$-cycloalkyl-méthoxy]-2 éthyles et $[(C_{3-6})$-cycloalcoxy]-2 éthyles.

Les sels d'addition pharmaceutiquement acceptables que peuvent former les composés (I) avec des acides font partie de l'invention.

Selon l'invention on peut préparer les composés (I) selon plusieurs méthodes:

1. Schéma 1

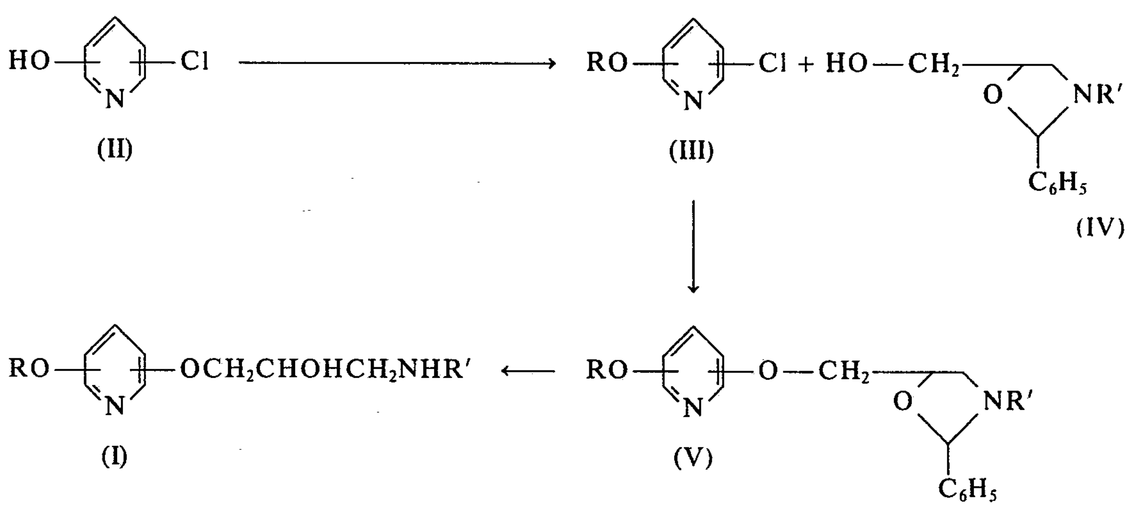

(II) (III) (IV) (I) (V)

Les composés de départ (II) sont décrits dans la littérature.

Les composés (III) sont obtenus par réaction entre le composé (II) et le tosylate de l'alcool ROH, dans un solvant tel que le diméthyl-formamide, le diméthoxyéthane ou le diglyme, à une température allant de 20 à 100°C.

Les oxazolidines (IV) sont connues et décrites dans la littérature. Elles peuvent exister sous forme optiquement active. En conséquence, pour préparer les composés (I) optiquement actifs, on peut faire réagir un composé (III) avec une oxazolidine (IV) optiquement active. La réaction entre le composé (III) et l'oxazolidine (IV) est effectuée dans un solvant tel que le diméthyl-formamide, le diméthoxyéthane ou le diglyme à une température de 20 à 150°C.

L'ouverture du cycle du composé (V) est effectuée en milieu acide, à une température allant de 0 à 80°C.

2. Schéma 2

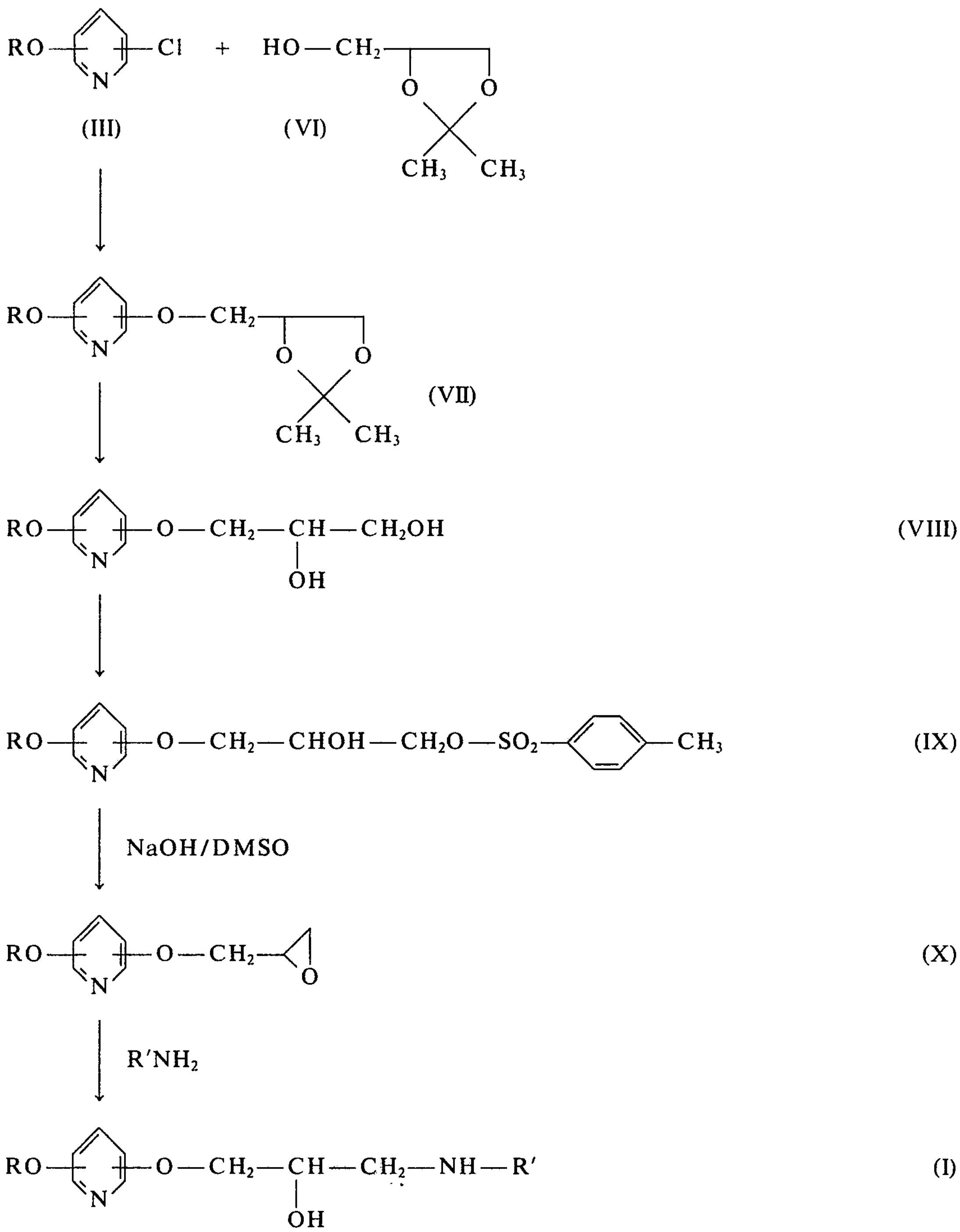


(III) (VI) (VII) (VIII) (IX) (X) (I)

3. Schéma 3

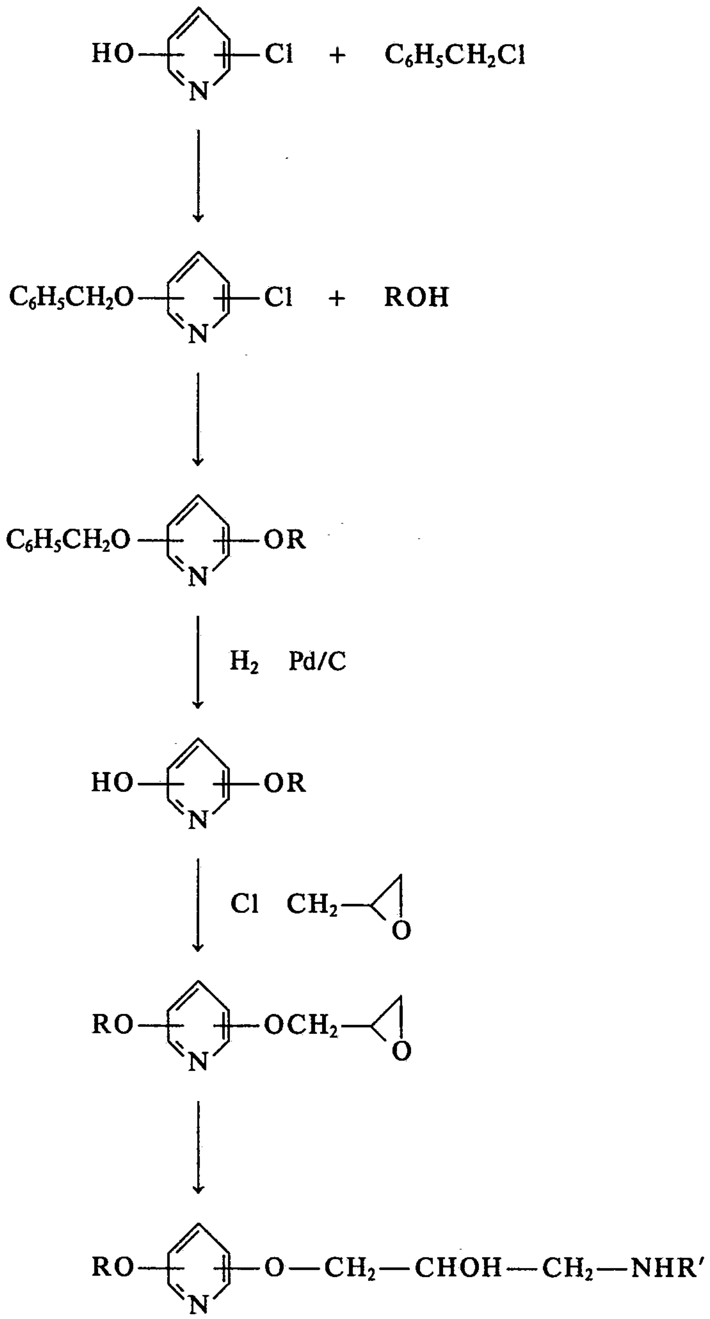


(XI)

(XII)

(XIII)

(X)

(I)

4. Schéma 4

[variante du schéma 2 à partir du composé (VIII)]

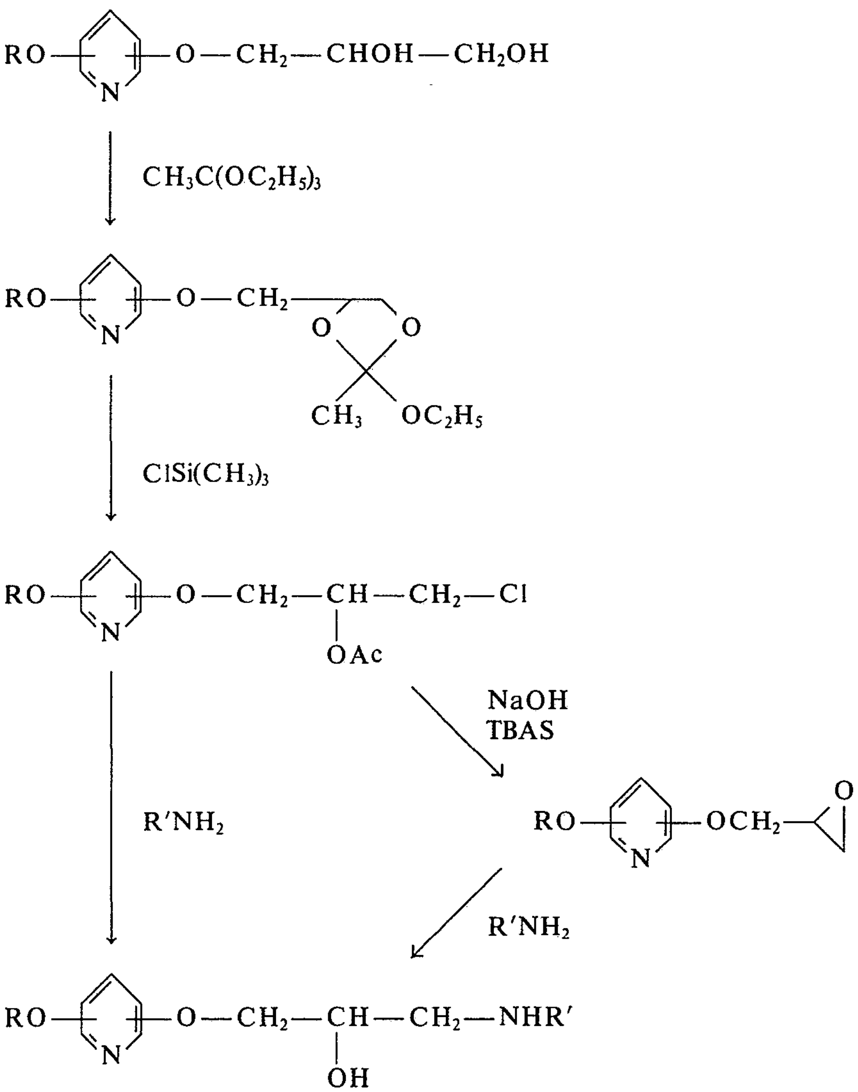


(VIII)

(XIV)

(XV)

(X)

(I)

La réaction entre le composé (III) et le composé (VI) est effectuée dans un solvant tel que le DMF, DME ou diglyme à une température allant de 20 à 150°C.

L'ouverture du cycle du composé (VII) est effectuée en milieu acide à une température allant de 10 à 80°C.

On estérifie le composé (VIII) par exemple, à l'aide d'un chlorure d'acide organique fort tel que le chlorure de méthane-sulfonyle, ou de méthyl-4phényl-sulfonyle. On conduit la réaction en présence d'une base inorganique, par exemple un carbonate alcalin ou un hydroxyde de métal alcalin ou alcalinoterreux ou d'une base organique telle que la pyridine ou la triethylamine dans un solvant tel que la pyridine ou le chloroforme et à une température d'environ −20 à +120°C.

L'epoxydation peut être réalisée suivant les méthodes classiques par traitement du composé (IX) par une base inorganique, par exemple un hydroxyde alcalin ou alcalinoterreux dans un solvant aprotique dipolaire tel que le DMF, HMPT ou DMSO, à une température allant de 20 à 100°C.

On peut également obtenir le composé de formule (X) en faisant réagir un composé (XIII) avec une épihalohydrine en présence d'une base telle qu'un hydroxyde ou hydrure alcalin, dans un solvant approprié tel que le DMF, DME ou diglyme, à une température allant de 20 à 150°C.

On peut également obtenir le composé (X) en faisant réagir un composé de formule (VIII) avec de l'orthoacétate de triéthyle puis avec du chlorure de triméthylsilyle pour obtenir le composé (XIV) puis le composé (XV). Ce dernier donne l'époxyde (X) par traitement avec une base telle que le sulfate de tertiobutylammonium en présence d'hydroxyde de sodium dans un solvant chloré tel que le dichlorométhane.

Les composés (X) ou (XV), par réaction, à une température de 20 à 150°C, avec les amines $R'NH_2$, avec ou sans solvant donnent les composés (I) cherchés.

Les exemples suivants illustrent l'invention. Les spectres IR et RMN ainsi que les analyses ont confirmé la structure des composés.

## Exemple 1

[(Cyclopropylméthoxy-2 ethoxy)-5 pyridinyl-2)oxy]-3 isopropylamino-1 propanol-2
et son fumarate neutre

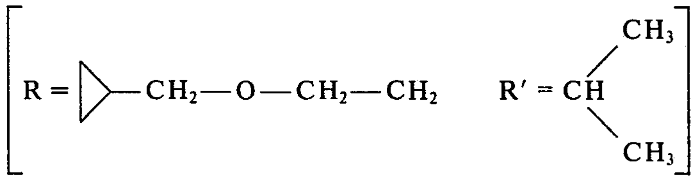

### 1. Chloro-2(cyclopropylméthoxy-2 éthoxy)-5 pyridine

On ajoute 12,9 g (0,1 mole) de chloro-2 hydroxy-5pyridine en solution dans 50 $cm^3$ de DMF sec, à une suspension de 4,8 g (0,1 mole) de NaH (à 50% dans de l'huile minérale) dans 200 $cm^3$ de DMF. Lorsque le dégagement d'hydrogène a cessé, on ajoute 27 g (0,1 mole) de tosylate de cyclopropylméthoxy-2 éthanol en solution dans 50 $cm^3$ de DMF puis on chauffe le mélange à 50—60°C pendant 5 h. On le refroidit puis le verse sur de l'eau, on extrait avec de l'éther et lave la phase organique avec de l'eau. Après séchage, filtration et évaporation de la phase éthérée on obtient une huile que l'on distille.

$Eb_{0,1}$ = 136°C

### 2. [(Cyclopropylméthoxy-2éthoxy)-5pyridinyl-2oxy]-3 isopropylamino-1propanol-2

On ajoute 6,6 g ($3,10^{-2}$ mole) d'hydroxyméthyl-5 isopropyl-3phényl-2 oxazolidine en solution dans 10 $cm^3$ de diglyme à une suspension de 1,5 g de NaH à 50%, dans 50 $cm^3$ de diglyme. On chauffe à 60—70°C pour achever la sodation, on refroidit à l'aide d'un bain d'eau glacée puis on ajoute 6,9 g ($3,10^{-2}$ mole) de chloro-2 (cyclopropylméthoxy-2 éthoxy)-5 pyridine en solution dans 10 $cm^3$ de diglyme, puis on porte le mélange à la température de 140°C pendant 3 à 4 heures.

On refroidit le mélange puis le verse sur de l'eau, et extrait avec de l'éther; on lave la phase éthérée avec de l'eau, séche sur $MgSO_4$, filtre et évapore.

On reprend le résidu huileux dans 100 $cm^3$ d'eau, on ajoute 20 $cm^3$ d'HCl concentré et on agite la solution pendant 30 minutes à la température ambiante. On extrait le benzaldéhyde formé plusieurs fois avec de l'éther, puis alcalinise la phase aqueuse avec de la soude et extrait de nouveau avec de l'éther. La phase organique est lavée avec de l'eau, séchée et évaporée. On obtient une huile à partir de laquelle on prépare le fumarate neutre dans un mélange acétone-éther.

F = 101—102°C

## Exemple 2

[(Cyclopropylméthoxy-2 éthoxy)-2 pyridinyl-5 oxy]-3 isopropylamino-1 propanol-2
et son fumarate neutre

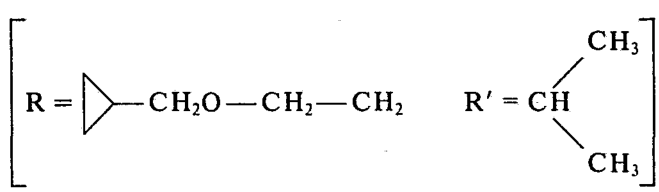

1. Chloro-2 benzyloxy-5 pyridine

On ajoute 12,9 g (0,1 mole) d'hydroxy-5 chloro-2 pyridine en solution dans 50 ml de DMF à une suspension de 4,8 g (0,1 mole) de NaH à 50% (préalablement lavé avec du toluène) dans 100 ml de DMF.

On agite jusqu'à cessation du dégagement d'hydrogène, refroidit à l'aide d'un bain glacé et introduit goutte à goutte 12,6 g (0,1 mole, 11,5 $cm^3$) de chlorure de benzyle. On chauffe à 60°C pendant une heure, laisse la nuit au repos puis verse le mélange sur de l'eau glacée et extrait avec de l'éther. On lave la phase éthérée avec de l'eau, sèche, filtre et évapore. On distille le produit.

$Eb_{0,1}$ = 135°C

2. (Cyclopropylméthoxy-2 éthoxy)-2 benzyloxy-5 pyridine

On ajoute 8,7 g (0,075 mole) de cyclopropylméthoxy-2 éthanol dans 25 $cm^3$ de diglyme à une suspension de 3,6 g (0,075 mole) de NaH à 50% (lavé avec du toluène). On chauffe à 60—70°C pour achever la sodation, refroidit le mélange au bain de glace et ajoute goutte à goutte 16,4 g (0,075 mole) de chlorure de benzyle en solution dans 50 $cm^3$ de diglyme puis chauffe le mélange à 100°C.

On porte le mélange à 130—140°C pendant 5 h., refroidit et verse sur de l'eau glacée. On extrait plusieurs fois avec de l'éther. On réunit les extraits organiques, les lave avec de l'eau, sèche, filtre et évapore.

On distille le produit.

$Eb_{0,1}$ = 180—190°C

3-(Cyclopropylméthoxy-2 éthoxy)-2 hydroxy-5 pyridine

On hydrogène dans l'appareil de Parr, à la température ambiante, sous 50 psi d'hydrogène, en présence de palladium sur charbon, 10 g (0,1/3 mole) du dérivé précédent en solution dans 100 $cm^3$ de méthanol et 1,9 $cm^3$ d'acide acétique.

Lorque l'absorption est terminée, on filtre le catalyseur et évapore le filtrat. On reprend le résidu d'évaporation avec de l'eau, alcalinise avec de la soude et extrait avec de l'éther.

On lave la phase éthérée avec de l'eau, on sèche, filtre et évapore. On recueille une huile.

4-(Cyclopropylméthoxy-2 éthoxy)-2 [(oxirannyl-2)méthoxy]-5 pyridine

On porte au reflux pendant 12 heures, 4,2 g ($2,10^{-2}$ mole) du pyridinol précédent et 5 g de $K_2CO_3$, 15 ml de

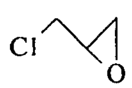

et 50 ml de $CH_3CN$.

On évapore à sec, reprend le résidu d'évaporation par un mélange eau-éther. On lave la phase éthérée avec de l'eau, sèche avec $MgSO_4$, filtre et évapore. On obtient une huile jaune que l'on utilise sans autre purification.

5-[(Cyclopropylméthoxy-2 éthoxy)-2 pyridinyl-5 oxy]-3 isopropylamino-1 propanol-2 et son fumarate neutre

On chauffe à la température du reflux, 5 g de l'époxyde précédent en solution dans 50 $cm^3$ d'isopropylamine.

On maintient le reflux pendant 24 heures puis on évapore à sec.

On chromatographie l'huile résiduelle sur une colonne de silice (20 g/g éluant $CHCl_3$/MeOH 95/5).

Après évaporation des fractions pures, on triture l'huile résiduelle dans de l'éther isopropylique puis filtre l'insoluble.

On évapore le filtrat à sec et recueille 3,1 g d'huile à partir de laquelle on prépare le fumarate dans de l'acétone. On recristallise le fumarate dans de l'acétone.

F = 106—108°C

## Exemple 3

(S) (—) [(Cyclopropylméthoxy-2 éthoxy)-5 pyridinyl-2 oxy]-3 t-butylamino-1 propanol-2 et son maléate

On ajoute 3,5 g (0,01 mole) de (S) phényl-2 t-butyl-3 hydroxyméthyl-5 oxazolidine en solution dans 10 $cm^3$ de diglyme à une suspension de 0,7 g (0,015 mole) d'hydrure de sodium à 50% dans 10 $cm^3$ de diglyme, puis, goutte à goutte, 3,4 g (0,015 mole) de (cyclopropyl-méthoxy-2 ethoxy)-5 chloro-2 pyridine. On chauffe le mélange à 130—140°C pendant 6 heures, le refroidit puis le verse dans de l'eau et on extrait avec de l'éther. On lave la phase éthérée avec de l'eau, la sèche ($MgSO_4$), filtre et évapore. On obtient une huile dont le maléate, recristallisé dans de l'acétone, fond à 164—165°C $[\alpha]_D = -10,7$ (c = 1,1 $CH_3OH$).

Dans le tableau suivant sont représentés les composés de l'invention préparés à titre d'exemples.

Tableau

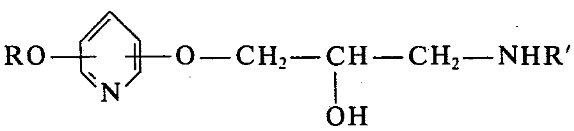

| Composé | Position de OR | Radical R | Position du groupe O ... NHR' | R' | F. (°C) |
|---|---|---|---|---|---|
| 1 racemate | 5 | 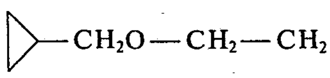 | 2 | 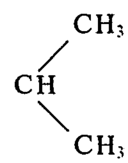 | 101—102 fumarate |
| 1 isomère(s) | | desgl. | 2 | desgl. | 115—117 maléate |
| 2 racemate | 5 | 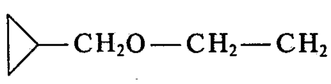 | 2 | 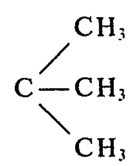 | 168—169 fumarate |
| 2 isomère(s) | | desgl. | 2 | desgl. | 164—165 maléate |
| 3 | 5 | 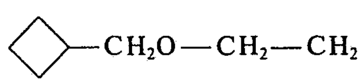 | 2 | 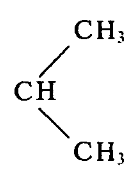 | 117—118 fumarate |
| 4 | 5 | 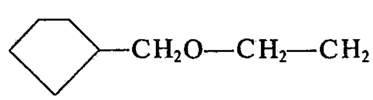 | 2 | 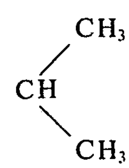 | 116—117 fumarate |
| 5 | 5 | 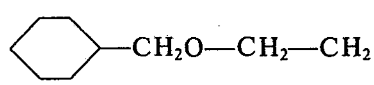 | 2 | 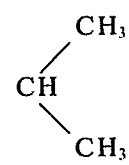 | 123 fumarate |

Tableau (suite et fin)

| Composé | Position de OR | Radical R | Position du groupe O ... NHR' | R' | F. (°C) |
|---|---|---|---|---|---|
| 6 | 5 | 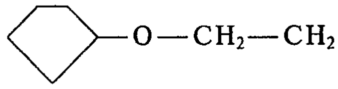 | 2 | 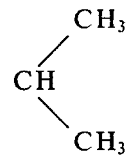 | 120–122 fumarate |
| 7 | 5 | 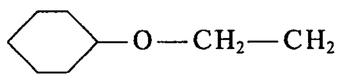 | 2 | 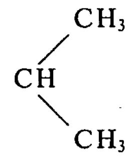 | 120–121 fumarate |
| 8 | 5 | $CH_2$=CH-$CH_2$-O-$CH_2$-$CH_2$ | 2 | 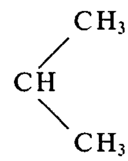 | 102–103 fumarate |
| 9 | 2 | 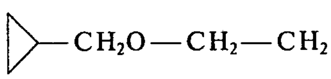 | 5 | 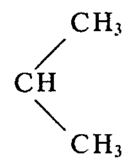 | 106–108 fumarate |

Les composés (I) de l'invention ont été soumis à une série d'essais pharmacologiques qui ont mis en évidence leurs propriétés intéressantes dans le domaine cardio-vasculaire.

La toxicité aigüe par voie orale et intraveineuse a été évaluée chez des souris mâles CDI, d'un poids moyen de 20 g. On a noté la mortalité sur une période de 5 jours suivant l'administration des composés. La dose létale 50% ($DL_{50}$) a été calculée selon Litchfield et Wilcoxon (J. Pharmacol. Exp. Ther 1944, 95, 99. La $DL_{50}$ va de 600 à 2000 mg/kg per os.

Etudes sur organes isolés: On a utilisé des oreillettes isolées prélevées sur des rats pesant 250 à 350 g, maintenues dans la solution de Moran (en g/l: NaCl = 7,02; KCl = 0,42; $CaCl_2$ = 0,24; $MgCl_2$ = 0,20; $NaHCO_3$ = 2,0; glucose = 1,8), oxygénée (95% $O_2$ — 5% $CO_2$) à la température de 31°C. On a étudié la tachycardie et l'augmentation de la force contractile provoquée par l'isoprénaline (courbe dose — réponse à l'agoniste) avant et après l'addition de l'antagoniste [composé (I) ou substance de référence] et on a calcule le $pA_2$ de chacun d'eux par la méthode de Arunlakshana et Schild (Brit. J. Pharmacol. 1959, 14, 48) le $pA_2$ représentant le logarithme de la concentration molaire d'antagoniste compétitif nécessitant une dose d'agoniste deux fois plus forte pour provoquer les mêmes réponses que celles obtenues en l'absence d'antagoniste. Le $pA_2$ des composés est compris entre 8 et 9.

Tous les composés (I) possèdent une activité inhibitrice vis-à-vis des effets cardiaques de l'isoprénaline mais non vis-à-vis des effets hypotenseurs de cette dernière: ce sont donc bien des agents de blocage sélectif des récepteurs $\beta_1$-adrénergiques, c'est-à-dire les récepteurs $\beta$-adrénergiques situés au niveau du coeur et non vis-à-vis des récepteurs $\beta_2$ adrénergiques situés au niveau des vaisseaux.

Les résultats qui précèdent montrent que les composés de l'invention sont utilisables en médecine humaine et vétérinaire, dans les maladies cardiovasculaires et, notamment, dans les affections coronariennes, les affections atteignant le myocarde et les troubles du rythme cardiaque.

L'invention comprend, par conséquent, toutes compositions pharmaceutiques renfermant les composés de formule générale (I) et leurs sels comme principes actifs en association avec tous excipients appropriés à leur administration par voie orale, ou rectale, ou parentérale. Ces compositions pharmaceutiques peuvent également contenir d'autres substances médicamenteuses avec lesquelles ces composés et leurs sels sont pharmaceutiquement et thérapeutiquement compatibles.

Pour l'administration par voie orale, on utilise toutes les formes pharmaceutiques appropriées à cette voie, c'est-à-dire les comprimés, dragées, gélules, capsules, cachets, solutions et suspensions buvables, la prise unitaire du principe actif pouvant varier entre 5 et 100 mg, et la dose quotidienne étant comprise entre 10 et 300 mg.

Pour l'administration par voie endorectale, on utilise des suppositoires renfermant 10 à 100 mg de principe actif et administré au malade à raison de 1 à 3 par 24 heures.

Pour l'administration par voie parentérale, on utilise des solutés injectables stabilisés et temponnés, préparés à l'avance ou extemporanément. La dose de principe actif par prise unitaire peut varier entre 1 et 10 mg, et la dose quotidienne est comprise entre 3 et 50 mg.

**Revendications your les Etats contractants: BE, CH, DE, FR, GB, IT, LI, LU, NL, SE**

1. Dérivés de pyridine, sous la forme de racémates ou d'antipodes lévogyres et dextrogyres, répondant à la formule (I)

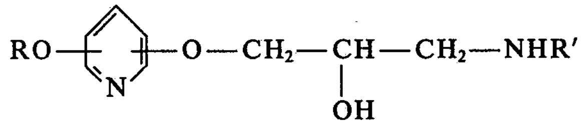

(I)

dans laquelle

- R représente un radical $R_1O(CH_2)_2$ — dans lequel
  $R_1$ est un radical $(C_{3-6})$cycloalkyle, un radical $(C_{3-6})$cycloalkyl-$(C_{1-4})$alkyle ou un radical allyle, et
- R' représente un radical isopropyle ou tertiobutyle,

ainsi que leurs sels d'addition aux acides pharmaceutiquement acceptables.

2. Dérivés selon la revendication 1, caractérisés par le fait que le groupe $-OCH_2-CHOH-CH_2-NHR'$ est en position 2 sur le cycle pyridinique.

3. Dérivés selon la revendication 2, caractérisés par le fait que le groupe $-OCH_2-CHOH-CH_2-NHR'$ est en position 2 et le groupe RO est en position 5 sur le cycle pyridinique.

4. Derivés selon l'une quelconque des revendications 1, 2 et 3, caractérisés par le fait que R est un radical [$(C_{3-6})$-cycloalkyl)méthoxy]-2 éthyle ou [$(C_{3-6})$-cycloalcoxy]-2 éthyle.

5. Le [(cyclopropylméthoxy-2 ethoxy)-5 pyridinyl-2) oxy]-3 isopropylamino-1 propanol-2 et ses sels.

6. Le [(cyclopentylméthoxy-2 éthoxy)-5 pyridinyl-2) oxy]-3 isopropylamino-1 propanol-2 et ses sels.

7. Le [(cyclohexylméthoxy-2 éthoxy)-5 pyridinyl-2) oxy]-3 isopropylamino-1 propanol-2 et ses sels.

8. Le [(cyclopentoxy-2 éthoxy)-5 pyridinyl-2) oxy]-3 isopropylamino-1 propanol-2 et ses sels.

9. Procédé de préparation des composés selon la revendication 1, procédé caractérisé en ce que l'on transforme une chlorohydroxy-pyridine de formule (II)

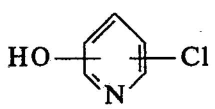

(II)

en chloro-RO-pyridine de formule (III)

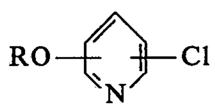

(III)

que l'on fait réagir avec une oxazolidine (IV)

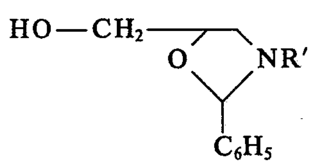

(IV)

et on ouvre le cycle du composé (V)

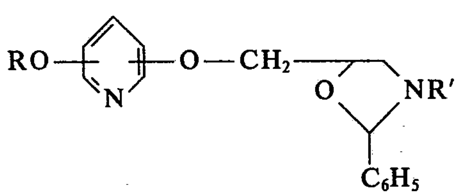

(V)

en milieu acide pour obtenir le composé (I)

10. Composition pharmaceutique, caractérisée en ce qu'elle contient un composé selon l'une quelconque des revendications 1 à 8 en association avec tout excipient approprié.

11. Médicament, caractérisé en ce qu'il contient un composé selon l'une quelconque des revendications 1 à 8.

**Revendication your l'Etat contractant: AT**

Procédé de préparation des dérivés de pyridine, sous la forme de racémates ou d'antipodes lévogyres et dextrogyres, répondant à la formule (I)

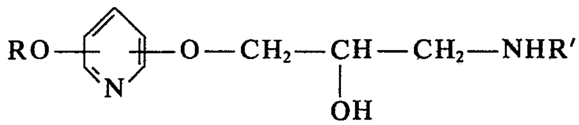

(I)

dans laquelle
R représente un radical $R_1O(CH_2)_2$ — dans lequel
$R_1$ est un radical $(C_{3-6})$cycloalkyle, un radical $(C_{3-6})$cycloalkyl-$(C_{1-4})$alkyle ou un radical allyle, et
R' représente un radical isopropyle ou tertiobutyle,
ainsi que de leurs sels d'addition aux acides pharmaceutiquement acceptables,
procédé caractérisé en ce que l'on transforme une chlorohydroxypyridine de formule (II)

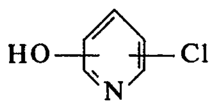

(II)

en chloro-RO-pyridine de formule (III)

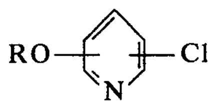

(III)

que l'on fait réagir avec une oxazolidine (IV)

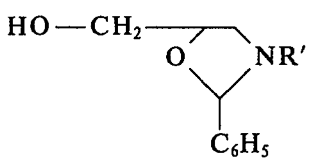

(IV)

et on ouvre le cycle du composé (V)

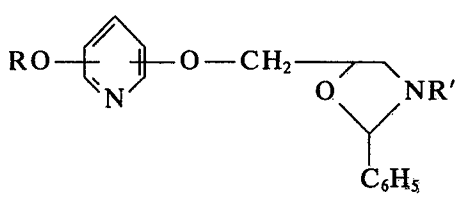

(V)

en milieu acide pour obtenir le composé (I)

**Patentansprüche für die Vertragsstaaten: BE, CH, DE, FR, GB, IT, LI, LU, NL, SE**

1. Pyridinderivate in Form der Racemate oder der rechts- und linksdrehenden Antipoden, entsprechend der Formel (I)

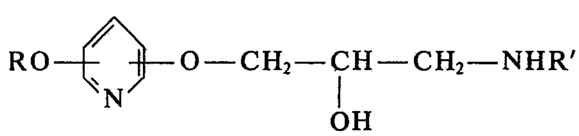

(I)

wobei

R eine Gruppe $R_1O(CH_2)_2$ bedeutet, bei welcher $R_1$ eine $(C_{3-6})$-Cycloalkylgruppe, eine $(C_{3-6})$-Cycloalkyl-$(C_{1-4})$-Alkylgruppe oder eine Allylgruppe ist, und
R′ eine Isopropyl- oder eine tertiäre Butylgruppe bedeutet,
sowie ihre Additionssalze mit pharmazeutisch verwendbaren Säuren.

2. Derivate nach Anspruch 1, dadurch gekennzeichnet, daß sich die Gruppe $-OCH_2-CHOH-CH_2-NHR'$ in 2-Stellung am Pyridinring befindet.

3. Derivate nach Anspruch 2, dadurch gekennzeichnet, daß sich die Gruppe $-OCH_2-CHOH-CH_2-NHR'$ in 2-Stellung und die Gruppe RO in 5-Stellung am Pyridinring befindet.

4. Derivate nach einem der Ansprüche 1, 2 und 3, dadurch gekennzeichnet, daß R eine 2-[$(C_{3-6})$-Cycloalkylmethoxy]-äthyl- oder eine 2-[$(C_{3-6})$-Cycloalkoxy]-äthyl-Gruppe ist.

5. 3-[5-(2-(Cyclopropylmethoxy)-äthoxy)-2-pyridinyl-oxy]-1-isopropylamino-propanol-(2) und seine Salze.

6. 3-[5-(2-(Cyclopentylmethoxy)-äthoxy)-2-pyridinyl-oxy]-1-isopropylamino-propanol-(2) und seine Salze.

7. 3-[5-(2-(Cyclohexylmethoxy)-äthoxy)-2-pyridinyl-oxy]-1-isopropylamino-propanol-(2) und seine Salze.

8. 3-[5-(2-(Cyclopentoxy)-äthoxy)-2-pyridinyl-oxy]-1-isopropylamino-propanol-(2) und seine Salze.

9. Verfahren zur Herstellung der Verbindungen nach Anspruch 1, dadurch gekennzeichnet, daß ein Chlorhydroxypyridin der Formel (II)

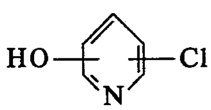

(II)

in ein Chlor-RO-Pyridin der Formel (III)

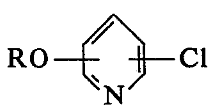

(III)

umgewandelt wird, welches mit einem Oxazolidin (IV)

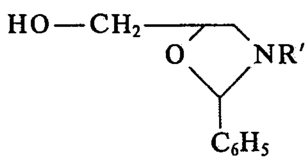

(IV)

zur Reaktion gebracht wird, und in saurem Milieu der Ring der Verbindung (V)

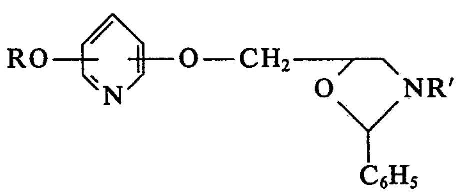

(V)

geöffnet wird, um Verbindung (I) zu erhalten.

10. Pharmazeutische Zubereitung, dadurch gekennzeichnet, daß sie eine Verbindung nach einem der Ansprüche 1 bis 8 in Verbindung mit irgendeinem geeigneten Exzipiens enthält.

11. Arzneimittel, dadurch gekennzeichnet, daß es eine Verbindung nach einem der Ansprüche 1 bis 8 enthält.

**Patentanspruch für den Vertragsstaat: AT**

Verfahren zur Herstellung von Pyridinderivaten in Form der Racemate oder der rechts- und linksdrehenden Antipoden, entsprechend der Formel (I)

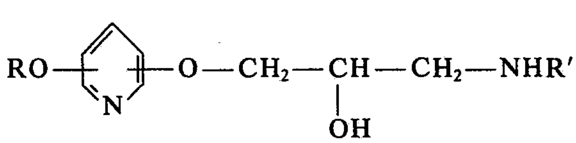

(I)

wobei
R eine Gruppe $R_1O(CH_2)_2$ bedeutet, bei welcher $R_1$ eine $(C_{3-6})$-Cycloalkylgruppe, eine $(C_{3-6})$-Cycloalkyl-$(C_{1-4})$-Alkylgruppe oder eine Allylgruppe ist, und
R' eine Isopropyl- oder eine tertiäre Butylgruppe bedeutet,
sowie ihre Additionssalze mit pharmazeutisch verwendbaren Säuren.
dadurch gekennzeichnet, daß ein Chlorhydroxypyridin der Formel (II)

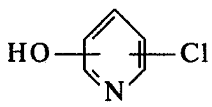
(II)

in ein Chlor-RO-Pyridin der Formel (III)

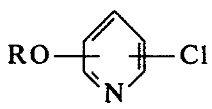
(III)

umgewandelt wird, welches mit einem Oxazolidin (IV)

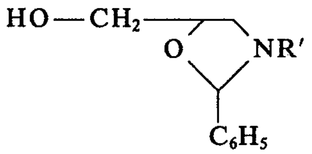
(IV)

zur Reaktion gebracht wird, und in saurem Milieu der Ring der Verbindung (V)

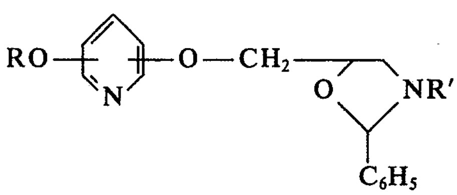
(V)

geöffnet wird, um Verbindung (I) zu erhalten.

**Claims for the Contracting states: BE, CH, DE, FR, GB, IT, LI, LU, NL, SE**

1. Pyridine derivatives, in the form of racemates or laevorotatory and dextrorotatory antipodes, corresponding to the formula (I)

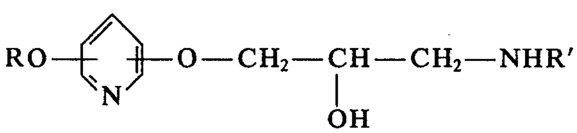
(I)

in which
R represents a radical $R_1O(CH_2)_2$—, in which $R_1$ is a $(C_{3-6})$-cycloalkyl radical, a $(C_{3-6})$-cycloalkyl-$(C_{1-4})$-alkyl radical or an allyl radical, and
R' represents an isopropyl or tert.-butyl radical,
and also their addition salts with pharmaceutically acceptable acids.
2. Derivatives according to Claim 1, characterised in that the group $-OCH_2-CHOH-CH_2-NHR'$ is in the 2-position on the pyridine ring.
3. Derivatives according to Claim 2, characterised in that the group $-OCH_2-CHOH-CH_2-NHR'$ is in the 2-position and the group RO is in the 5-position on the pyridine ring.
4. Derivatives according to any one of Claim 1, 2 and 3, characterised in that R is a 2-[$(C_{3-6})$-cycloalkylmethoxy]-ethyl or 2-[$(C_{3-6})$-cycloalkoxy]-ethyl radical.
5. 3-[5-(2-cyclopropylmethoxy-ethoxy)-pyridin-2-yl-oxy]-1-isopropylamino-propan-2-ol and its salts.
6. 3-[5-(2-cyclopentylmethoxy-ethoxy)-pyridin-2-yl-oxy]-1-isopropylamino-propan-2-ol and its salts.
7. 3-[5-(2-cyclohexylmethoxy-ethoxy)-pyridin-2-yl-oxy]-1-isopropylamino-propan-2-ol and its salts.
8. 3-[5-(2-cyclopentoxy-ethoxy)-pyridin-2-yl-oxy]-1-isopropylamino-propan-2-ol and its salts.

9. Process for the preparation of the compounds according to claim 1, which process is characterised in that a chlorohydroxypyridine of the formula (II)

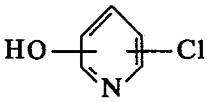

(II)

is converted to a chloro-RO-pyridine of the formula (III)

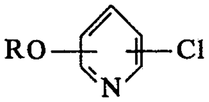

(III)

which is reacted with an oxazolidine (IV)

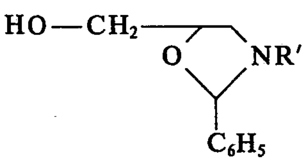

(IV)

and the ring of the compound (V)

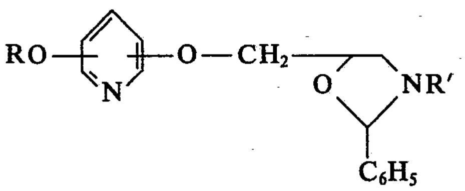

(V)

is opened in an acid medium in order to obtain the compound (I).

10. Pharmaceutical composition, characterised in that it contains a compound according to any one of claims 1 to 8, in association with any suitable excipient.

11. Medicament, characterised in that it contains a compound according to any one of claims 1 to 8.

**Claim for the Contracting state: AT**

Process for the preparation of pyridine derivatives, in the form of racemates or laevorotatory and dextrorotatory antipodes, corresponding to the formula (I)

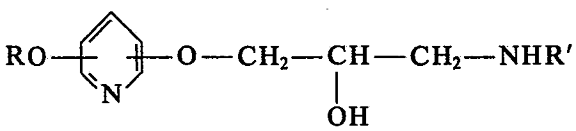

(I)

in which
R represents a radical $R_1O(CH_2)_2-$, in which $R_1$ is a $(C_{3-6})$-cycloalkyl radical, a $(C_{3-6})$-cycloalkyl-$(C_{1-4})$-alkyl radical or an allyl radical, and
R' represents an isopropyl or tert.-butyl radical, and also their addition salts with pharmaceutically acceptable acids which process is characterised in that a chlorohydroxypiridine of the formula (II)

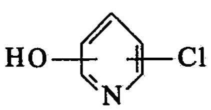

(II)

is converted to a chloro-RO-pyridine of the formula (III)

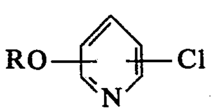

(III)

which is reacted with an oxazolidine (IV)

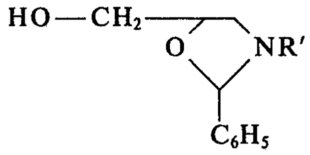

(IV)

and the ring of the compound (V)

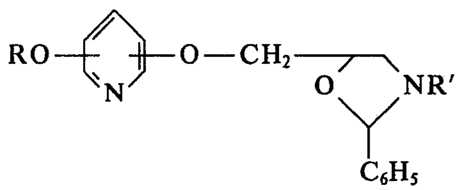

(V)

is opened in an acid medium in order to obtain the compound (I).